# EUROPÄISCHE PATENTSCHRIFT

(11) **EP 3 544 988 B1**
(45) Veröffentlichungstag und Bekanntmachung des Hinweises auf die Patenterteilung: **26.02.2020**
(21) Anmeldenummer: 17791596.4
(22) Anmeldetag: 10.10.2017
(51) Int. Cl.: C07F 17/00, C07F 7/08, C07C 15/44, C07F 7/18

(54) **INHIBIERTE EDELMETALLFREIE HYDROSILYLIERBARE MISCHUNG**
INHIBITED NOBLE-METAL-FREE MIXTURE THAT CAN BE HYDROSILYLATED
MÉLANGE HYDROSILYLABLE EXEMPT DE MÉTAUX NOBLES INHIBÉ

(43) Veröffentlichungstag der Anmeldung: 02.10.2019
(73) Patentinhaber: Wacker Chemie AG, 81737 München (DE)
(72) Erfinder: FRITZ-LANGHALS, Elke, 85521 Ottobrunn (DE)
(74) Vertreter: Mai, Marit
(86) Internationale Anmeldenummer: PCT/EP2017/075824
(87) Internationale Veröffentlichungsnummer: WO 2019/072378

(56) Entgegenhaltungen:
- DE-A1-102016 205 526

## Beschreibung

Die Erfindung betrifft eine hydrosilylierbare Mischung, die eine Verbindung mit kationischer Si(II)-Gruppierung als Katalysator enthält und ein Verfahren zur Hydrosilylierung der Mischung durch Erwärmen.

Die Addition von Hydrosiliciumverbindungen an Alkene und Alkine spielt in der Technik eine wichtige Rolle. Diese als Hydrosilylierung bezeichnete Reaktion wird sehr häufig zur Vernetzung von Siloxanen (Elastomervernetzung) oder zur Einführung von funktionellen Gruppen in Silane oder Siloxane eingesetzt. Die Hydrosilylierungen werden in der Technik in der Regel unter Verwendung von Edelmetall-Komplexen in Kombination mit Inhibitoren, häufig auf Basis von Alkinen, durchgeführt, die die Hydrosilylierung bei Umgebungstemperaturen blockieren. Hierdurch ist es möglich, die Komponenten einschließlich des Katalysators homogen zu mischen, ohne dass hierbei bereits eine Hydrosilylierung einsetzt. Der Hydrosilylierungsvorgang kann dann durch Temperaturerhöhung ausgelöst werden. Ein weiterer Vorteil ist, dass das Mischen zeitlich getrennt vom Hydrosilylierungsvorgang stattfinden und gezielt gesteuert werden kann, einkomponentige Systeme können allein durch Temperaturerhöhung umgesetzt werden.

Ein Nachteil der Edelmetall-katalysierten Hydrosilylierung ist der hohe Preis der Edelmetalle, welche weltweit nur begrenzt zur Verfügung stehen und nicht vorhersehbaren und nicht beeinflussbaren Preisschwankungen ausgesetzt sind. Außerdem ist es häufig nicht möglich oder nicht wirtschaftlich, die Edelmetalle zurückzugewinnen. Ein Hydrosilylierungskatalysator, der frei von Edelmetall ist, ist daher von großem technischem Interesse.

In jüngster Zeit wurden beispielsweise von Chirik et al. in Science 2012, 335, 567, ACS Catalysis 2016, 6, 2632 und ACS Catalysis 2016, 6, 4105 Übergangsmetallkomplexe, v.a. des Eisens, Kobalts und Nickels als Alternative vorgeschlagen, jedoch zeigen sie eine unzureichende Stabilität, so dass verhältnismäßig große Mengen eingesetzt werden müssen, was häufig zu Verfärbungen führt. Ein noch wesentlich gravierenderer Nachteil dieser Systeme ist jedoch, dass bislang keine inhibierten Systeme existieren, die es ermöglichen, die Hydrosilylierung während des Mischvorgangs zu unterdrücken und durch Temperaturerhöhung gezielt zu starten.

DE 10 2016 205526 A1 offenbart hydrosilylierbare Mischungen und Verfahren zur Hydrosilylierung mit Triethylsilan oder Dimethylphenylsilan oder Pentamethyldisiloxan, die eine Verbindung mit kationischer Si (II)-Gruppierung als Katalysator (π-Me₅C₅)Si⁺B(C₆F₅)₄⁻ enthalten.

Die Aufgabe der vorliegenden Erfindung bestand daher darin, eine Edelmetall-freie hydrosilylierbare Mischung bereitzustellen, welche bei Umgebungstemperatur über einen längeren Zeitraum stabil ist und deren Umsetzung durch Temperaturerhöhung ausgelöst werden kann.

Gegenstand der Erfindung ist eine Mischung **M** enthaltend
Verbindung **A,** die mindestens ein direkt an Si gebundenes Wasserstoffatom enthält,
Verbindung **B,** die mindestens eine Kohlenstoff-Kohlenstoff-Mehrfachbindungen enthält,
Verbindung **C,** die mindestens eine kationische Si(II)-Gruppierung enthält und
Verbindung **D,** die mindestens eine direkt an Silicium gebundene Alkoxygruppierung enthält, wobei das molare Verhältnis, der Verbindungen A und B bezogen auf die vorhandenen Si-H bzw. die ungesättigten Kohlenstoffgruppierungen mindestens 1:100 und höchstens 100:1 beträgt, wobei ferner das molare Verhältnis zwischen der Verbindung C und den in der Verbindung A vorhandenen Si-H-Gruppierungen mindestens 1:10⁷ und höchstens 1:1 beträgt und wobei ferner das molare Verhältnis der Verbindungen C und D bezogen auf die vorhandenen kationischen Si(II)-Gruppierungen der Verbindung C zu den direkt an Silicium gebundenen Alkoxygruppierungen der Verbindung D mindestens 1:1 und höchstens 1:200 beträgt.

Überraschend konnte gezeigt werden, dass die durch Verbindung **C** als nichtmetallischem Hydrosilylierungskatalysator katalysierte Hydrosilylierungsreaktion durch den Zusatz von Alkoxysiliciumverbindungen **D** insbesondere bei Umgebungstemperatur vollständig unterdrückt und durch Temperaturerhöhung ausgelöst werden kann.

Alkoxysiliciumverbindung **D** kann somit als Inhibitor für die Hydrosilylierungsreaktion mit dem nichtmetallischem Hydrosilylierungskatalysator **C** eingesetzt werden.

Die Verbindung **A** mit mindestens einem direkt an Si gebundenen Wasserstoffatom weist bevorzugt die allgemeine Formel I

**R¹R²R³Si-H** (I)

auf, wobei die Reste **R¹, R²** und **R³** unabhängig voneinander die Bedeutung Wasserstoff, Halogen, Silyloxyrest oder Kohlenwasserstoffrest haben, wobei jeweils einzelne Kohlenstoffatome durch Sauerstoffatome, Siliciumatome, Stickstoffatome, Halogen, Schwefel oder Phosphoratome ersetzt sein können.

Besonders bevorzugt bedeuten die Reste **R¹, R²** und **R³** unabhängig voneinander Wasserstoff, Halogen, unverzweigten, verzweigten, linearen, acyclischen oder cyclischen, gesättigten oder einfach oder mehrfach ungesättigten C1-C20-Kohlenwasserstoffrest, wobei einzelne Kohlenstoffatome durch Sauerstoff, Halogen, Stickstoff oder Schwefel ersetzt sein können, oder Silyloxyrest der allgemeinen Formel II

**(SiO**_{4/2}**)**ₐ**(R^{x}SiO**_{3/2}**)**_{b}**(R^{x}**₂**SiO**_{2/2}**)**_{c}**(R^{x}**₃**SiO**_{1/2}**)**_{d}⁻ **(II)**

in der
**R^{x}** unabhängig voneinander Wasserstoff, Halogen, unverzweigten, verzweigten, linearen, acyclischen oder cyclischen, gesättigten oder einfach oder mehrfach ungesättigten C1-C20-Kohlenwasserstoffrest bedeuten, wobei einzelne Kohlenstoffatome durch Sauerstoff, Halogen, Stickstoff oder Schwefel ersetzt sein können,
**a, b, c** und **d** unabhängig voneinander ganzzahlige Werte von 0 bis 100000 bedeuten, wobei die Summe aus **a, b, c** und **d** zusammen mindestens den Wert 1 annimmt.

Ganz besonders bevorzugt bedeuten die Reste **R¹, R²** und **R³** unabhängig voneinander Wasserstoff, Chlor, C1-C3-Alkyl- oder Alkylenrest, Phenylrest oder Silyloxyrest der allgemeinen Formel II, in der **R^{x}** unabhängig voneinander Wasserstoff, Chlor, C1-C6 Alkyl oder Alkylen oder Phenyl bedeuten.

Besonders bevorzugte Reste **R¹, R²** und **R³** sind die Reste Methyl, Ethyl, Propyl, Phenyl, Chlor oder Silyloxyrest, insbesondere der allgemeinen Formel II.

Besonders bevorzugte Reste **R^{x}** sind die Reste Methyl, Ethyl, Propyl, Phenyl und Chlor.

Beispiele für Verbindungen **A** der allgemeinen Formel (I) sind die folgenden Silane (Ph = Phenyl, Me = Methyl, Et = Ethyl): Me₃SiH, Et₃SiH, Me₂PhSiH, MePh₂SiH, Me₂ClSiH, Et₂ClSiH, MeCl₂SiH, Cl₃SiH, und die folgenden Siloxane:
HSiMe₂-O-SiMe₂H, Me₃Si-O-SiHMe-O-SiMe₃,
H-SiMe₂-(O-SiMe₂)ₘ-O-SiMe₂-H mit m = 1 bis 20000,
Me₃Si-O-(SiMe₂-O)ₙ(SiHMe-O)ₒ-SiMe₃ mit n = 1 bis 20000 und o = 1 bis 20000.

Die Verbindung **A** kann auch eine Mischung verschiedener Verbindungen, insbesondere der allgemeinen Formeln I sein, bei denen gegebenenfalls die Reste **R¹, R²** und **R³** verschiedene Reste der allgemeinen Formel II sein können.

Die Verbindungen **B** mit mindestens einer Kohlenstoff-Kohlenstoff-Mehrfachbindung werden vorzugsweise ausgewählt aus Verbindungen mit mindestens einer Kohlenstoff-Kohlenstoff-Doppelbindung der allgemeinen Formel IIIa

R⁴R⁵C=CR⁶R⁷ **(IIIa)**,

und aus Verbindungen mit mindestens einer Kohlenstoff-Kohlenstoff-Dreifachbindung der allgemeinen Formel IIIb

R⁸C≡CR⁹ **(IIIb),**

wobei
**R**⁴, **R⁵, R⁶, R⁷, R⁸** und **R⁹** unabhängig voneinander linearen, verzweigten, acyclischen oder cyclischen, gesättigten oder einfach oder mehrfach ungesättigten C1-C20-Kohlenwasserstoffrest bedeuten, wobei einzelne Kohlenstoffatome durch Silicium, Sauerstoff, Halogen, Stickstoff, Schwefel oder Phosphor ersetzt sein können.

Es können auch Gemische der Verbindungen der allgemeinen Formel IIIa und IIIb vorliegen.

Besonders bevorzugt bedeuten die Reste **R⁴, R⁵, R⁶, R⁷, R⁸** und **R⁹** unabhängig voneinander Wasserstoff, linearen, verzweigten, acyclischen oder cyclischen, gesättigten oder einfach oder mehrfach ungesättigten C1-C20-Kohlenwasserstoffrest, welcher durch eine oder mehrere Heteroatomgruppierungen, insbesondere die Gruppierungen Halogen, insbesondere Chlor, Nitril, Alkoxy, COO**R^{z}**, O-CO-**R^{z}**, NH-CO-**R^{z}**, O-CO-O**R^{z}** substituiert sein kann, wobei **R^{z}** unabhängig voneinander Wasserstoff, Chlor, C1-C6 Alkyl oder Alkylen oder Phenyl bedeutet.

Bevorzugt bedeutet einer oder mehrere Reste **R**⁴ bis **R**⁹ Wasserstoff.

Ganz besonders bevorzugt sind **R**⁴ und **R**⁵ oder **R**⁶ und **R**⁷ Wasserstoff.

Beispiele für Verbindungen **B** sind Ethylen, Propylen, 1-Butylen, 2-Butylen, Cyclohexen,
Styrol, α-Methylstyrol, 1,1-Diphenylethylen, cis-Stilben, trans-Stilben,
Allylchlorid, Acrylnitril, Allylglycidylether,
Vinyl-SiMe₂-[O-SiMe₂]ₙ-SiMe₂-Vinyl mit n = 0 bis 10000, Me₃Si-[O-SiMeVinyl]ₒ-[O-SiMe₂]ₚ-SiMe₃ mit o = 1 bis 1000 und p = 0 bis 1000 , Acetylen, Propin, 1-Butin, 2-Butin, Phenylacetylen und Diphenylacetylen.

Die Verbindungen **A** und **B** können auch durch eine oder mehrere chemische Bindungen miteinander verbunden sein, d.h. sie können beide in einem Molekül vorliegen.

Die Verbindung **C** enthält eine oder mehrere kationische Si(II) Gruppierungen.

Bevorzugt ist die Verbindung **C** eine kationische Si(II)-Verbindung der allgemeinen Formel IV

**([Si(II)Cp]**⁺**)**ₐ**X**^{a**-**} **(IV)**

worin
**Cp** einen π-gebundenen Cyclopentadienylrest der allgemeinen Formel V, welcher mit den Resten **R^{y}** substituiert ist, 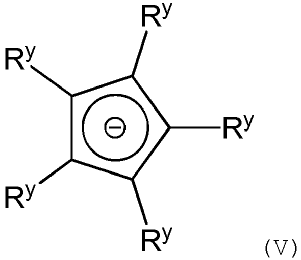 bedeutet.

Unter Cyclopentadienylrest Cp ist das Cyclopentadienyl-Anion zu verstehen, welches aus einem einfach negativ geladenen, aromatischen Fünfringsystem C₅R^{y}₅⁻ besteht.

**R^{y}** sind beliebige einwertige Reste oder mehrwertige Reste, die zur Bildung anellierter Ringe auch miteinander verbunden sein können.

Die Reste **R^{y}** bedeuten unabhängig voneinander bevorzugt Wasserstoff, lineare oder verzweigte, acyclische oder cyclische, gesättigte oder ein- oder mehrfach ungesättigte C1-C20 Alkyl- oder Aryl-, besonders bevorzugt C1-C3 Alkyl-, ganz besonders bevorzugt Methylreste.

Beispiele für Reste **R^{y}** sind Alkylreste, wie der Methyl-, Ethyl-, n-Propyl-, iso-Propyl-, n-Butyl-, sec-Butyl-, iso-Butyl-, tert.-Butyl-, n-Pentyl-, sec-Pentyl, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,4,4-Trimethylpentylrest; Nonylreste, wie der n-Nonylrest; Decylreste, wie der n-Decylrest; Dodecylreste, wie der n-Dodecylrest; Hexadecylreste, wie der n-Hexadecylrest; Octadecylreste, wie der n-Octadecylrest; Cycloalkylreste, wie der Cyclopentyl-, Cyclohexyl-, Cycloheptylrest und Methylcyclohexylrest; Arylreste, wie der Phenyl-, Naphthyl-, Anthryl- und Phenanthrylrest; Alkarylreste, wie der o-, m- und p-Tolyl-, Xylyl-, Mesitylenyl- und o-, m- und p-Ethylphenylrest; und Alkarylreste, wie der Benzylrest, der α- und der β-Phenylethylrest.

Weitere Beispiele für Verbindungen **C** sind die folgenden kationischen Si(II)-Verbindungen: 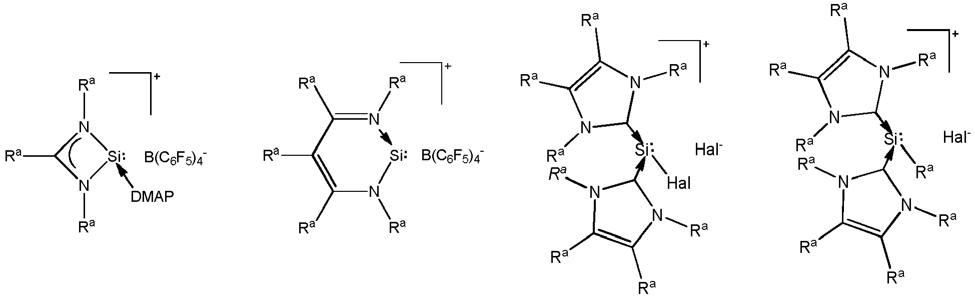 deren Herstellung in So et al, Chem. Eur. J. 2013, 19, 11786, Driess et al., Angew. Chem. Int. Ed. 2006, 45, 6730, Filippou, Angew. Chem. Int. Ed. 2013, 52, 6974, Sasamori et al, Chem. Eur. J. 2014, 20, 9246 und in Inoue et al., Chem. Commun. 2014, 50, 12619 beschrieben ist (DMAP = Dimethylaminopyridin).

In den vorstehenden Formeln bedeuten **R^{a}** Kohlenwasserstoffreste. Bevorzugt bedeuten die Reste **R^{a}** unabhängig voneinander Alkylrest, insbesondere C1-C20- Alkylrest oder substituierten oder unsubstituierten Phenylrest, besonders bevorzugt verzweigten Alkylrest oder 2,6-dialkylierten Phenylrest. **Hal** bedeutet Halogen, bevorzugt Chlor, Brom oder Jod. Beispiele für Reste **R^{a}** sind Methyl, Isopropyl, *tert*.-Butyl, 2,6-Diisopropylphenyl oder 2,4,6-Triisopropylphenyl.

**X^{a-}** bedeutet ein beliebiges **a** wertiges Anion, welches unter den Reaktionsbedingungen einer Hydrosilylierung mit dem kationischen Silicium(II)zentrum nicht reagiert. Es kann sowohl anorganisch als auch organisch sein. Vorzugsweise hat **a** die Werte 1, 2, oder 3, insbesondere 1.

**X⁻** bedeutet bevorzugt Halogen oder ein komplexes Anion wie BF₄⁻, ClO₄⁻, AlZ₄⁻, MF₆⁻ mit Z = Halogen und M = P, As oder Sb, oder Tetraarylboratanion, wobei der Arylrest bevorzugt Phenyl oder fluoriertes oder mit Perfluoralkylresten substituiertes Phenyl bedeutet, einwertiges polyedrisches Anion, wie z.B. Carboratanion, oder Alkoxy- und Aryloxymetallation.

Beispiele für Anionen **X⁻** sind Tetrachlorometallate [MCl₄]⁻ mit M = Al, Ga, Tetrafluoroborate [BF₄]⁻, Hexafluorometallate [MF₆]⁻ mit M = As, Sb, Ir, Pt, Perfluoroantimonate [Sb₂F₁₁]⁻, [Sb₃F₁₆]⁻ und [Sb₄F₂₁]⁻, Triflat (= Trifluoromethanesulfonat) [OSO₂CF₃]⁻, Tetrakis(trifluormethyl)borat [B(CF₃)₄]⁻, Tetrakis(pentafluorophenyl)metallate [M(C₆F₅)₄]⁻ mit M = B, Al, Ga, Tetrakis(pentachlorophenyl)borat [B(C₆Cl₅)₄]⁻, Tetrakis[(2,4,6-trifluoromethyl(phenyl)]borat {B[C₆H₂(CF₃)₃]}⁻, Hydroxybis[tris(pentafluorophenyl)borat]{HO[B(C₆F₅)₃]₂}⁻, *Closo-*Carborate [CHB₁₁H₅Cl₆]⁻, [CHB₁₁H₅Br₆]⁻, [CHB₁₁(CH₃)₅Br₆]⁻, [CHB₁₁F₁₁]⁻ , [C(Et)B₁₁F₁₁]⁻, [CB₁₁(CF₃)₁₂]⁻ und B₁₂Cl₁₁N(CH₃)₃]⁻, Tetra(perfluoroalkoxy)aluminat [Al(OR^{PF})₄]⁻, Tris(perfluoroalkoxy)fluoroaluminat [FAl(OR^{PF})₃]⁻, Hexakis(oxypentafluorotellur)antimonat [Sb(OTeF₅)₆]⁻.

Eine Übersicht über besonders bevorzugte komplexe Anionen **X⁻** findet sich z.B. in Krossing et. al., Angew. Chem. 2004, 116, 2116.

Die Herstellung der kationischen Si(II)-Verbindung der allgemeinen Formel IV kann durch Zugabe einer Säure H⁺X⁻ zu der Verbindung Si(II)Cp₂, durch die einer der anionischen Cp-Reste in protonierter Form abgespalten wird, erfolgen:

Si(II)Cp₂ + H⁺X⁻ -> Si(II)⁺Cp X⁻ + CpH

Das Anion **X**⁻ der Säure HX bildet dann das Gegenion der kationischen Silicium(II)-Verbindung.

Eine Herstellungsmethode für die kationische Si(II) Verbindung der allgemeinen Formel II ist in Science 2004, 305, S. 849-851, beschrieben:

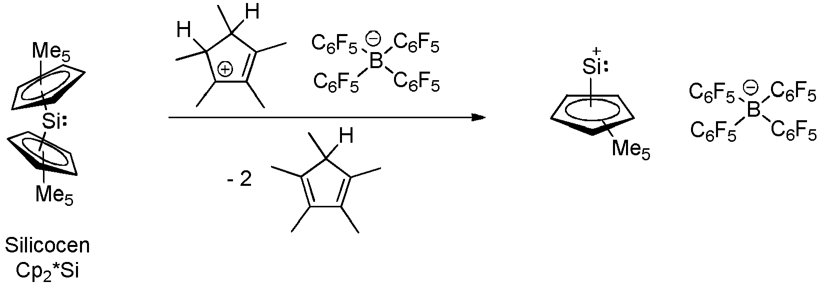

Die Bildung der kationischen Si(II)-Verbindung der allgemeinen Formel IV erfolgt dort mit Hilfe der Säure (Cp*H₂)⁺ B(C₆F₅)₄⁻ (Cp* = Pentamethylcyclopentadienyl). In diesem Fall wird die Verbindung der Formel IV mit dem Gegenion X⁻ = B(C₆F₅)₄⁻ erhalten, welches sehr gut kristallisierbar ist und daher besonders leicht isoliert werden kann. Die Verbindung der allgemeinen Formel IV kann jedoch auch durch Zugabe anderer Bronstedt-Säuren erzeugt werden, wobei Säuren bevorzugt sind, deren Anionen den oben angegebenen Anforderungen der schwachen Koordination entsprechen.

Die Verbindung **D** mit mindestens einer direkt an Silicium gebundenen Alkoxygruppierung hat bevorzugt die allgemeine Formel VI

**R¹³R¹⁴R¹⁵Si-O-CH₂-R¹⁶** **(VI)**

wobei die Reste **R¹³, R¹⁴** und **R¹⁵** unabhängig voneinander die Bedeutung Wasserstoff, Halogen, Silyloxyrest, vorzugsweise der vorstehenden allgemeinen Formel II, oder Kohlenwasserstoffrest haben, wobei jeweils einzelne Kohlenstoffatome durch Sauerstoffatome, Halogen, Schwefel oder Phosphoratome ersetzt sein können und
**R¹⁶** die Bedeutung Wasserstoff oder Kohlenwasserstoffrest hat, bei dem einzelne nicht benachbarte Kohlenstoffatome durch Sauerstoffatome, Silicium-, Halogen-, Schwefel- oder Phosphoratome ersetzt sein können.

Besonders bevorzugt bedeuten die Reste **R¹³, R¹⁴** und **R¹⁵** unabhängig voneinander Wasserstoff, Halogen, unverzweigten, verzweigten, linearen, acyclischen oder cyclischen, gesättigten oder einfach oder mehrfach ungesättigten C1-C20-Kohlenwasserstoffrest oder unverzweigten, verzweigten, linearen oder cyclischen, gesättigten oder einfach oder mehrfach ungesättigten C1-C20-Kohlenwasserstoffoxyrest, wobei einzelne Kohlenstoffatome durch Sauerstoff, Halogen oder Schwefel ersetzt sein können, oder Silyloxyrest der allgemeinen Formel **II,** in der
**R^{x}** die vorstehenden Bedeutungen und bevorzugten Bedeutungen aufweist.

Besonders bevorzugt bedeutet **R¹⁶** Wasserstoff, unverzweigten, verzweigten, linearen, acyclischen oder cyclischen, gesättigten oder einfach oder mehrfach ungesättigten C1-C20-Kohlenwasserstoffrest oder unverzweigten, verzweigten, linearen oder cyclischen, gesättigten oder einfach oder mehrfach ungesättigten C1-C20-Kohlenwasserstoffoxyrest, wobei einzelne Kohlenstoffatome durch Sauerstoff, Halogen oder Schwefel ersetzt sein können, oder Silyloxyrest der allgemeinen Formel II, in der
**R^{x}** die vorstehenden Bedeutungen und bevorzugten Bedeutungen aufweist.

**a, b, c** und **d** bedeuten unabhängig voneinander ganzzahlige Werte von 0 bis 100000, wobei die Summe aus **a, b, c** und **d** zusammen mindestens den Wert 1 annimmt.

Bevorzugt hat der Silyloxyrest der allgemeinen Formel **II** ein Molekulargewicht von unter 10000, besonders bevorzugt von unter 5000 und ganz besonders bevorzugt von unter 1000 Dalton.

Ganz besonders bevorzugt bedeuten die Reste **R**¹³, **R**¹⁴ und **R**¹⁵ unabhängig voneinander Wasserstoff, Chlor, C1-C3-Alkyl- oder Alkylenrest, Phenylrest oder Silyloxyrest der allgemeinen Formel **II,** in der **R^{x}** unabhängig voneinander Wasserstoff, Chlor, C1-C6 Alkyl oder Alkylen oder Phenyl bedeuten.

Ganz besonders bevorzugt bedeutet **R¹⁶** Wasserstoff, C1-C6-Alkyl- oder Alkylenrest oder Phenylrest.

Besonders bevorzugte Reste **R**¹³, **R**¹⁴ und **R**¹⁵ sind die Reste Methyl, Ethyl, Propyl, Phenyl, Chlor oder Silyloxyrest, insbesondere der allgemeinen Formel **II.**

Besonders bevorzugte Reste **R**¹⁶ sind die Reste Methyl, Ethyl, Propyl, Butyl oder Pentyl.

Besonders bevorzugte Reste **R**^{x} sind die Reste Methyl, Ethyl, Propyl, Phenyl und Chlor.

Beispiele für Verbindungen **D** der allgemeinen Formel **VI** sind die folgenden Silane (Ph = Phenyl, Me = Methyl, Et = Ethyl): Me₃SiOEt, Me₃SiOMe, Et₃SiOEt, Et₃SiOMe, Me₂PhSiOEt, Me₂PhSiOMe, MePh₂SiOEt, Me₃SiO-CH₂-CH₂-OSiMe₃, Me₃SiO-(CH₂)₃-OSiMe₃, Me₂Si(OEt)₂, Me₂Si(OMe)₂, MeSi(OEt)₃, MeSi(OMe)₃, Si(OEt)₄, MeSi(OMe)₄ und die folgenden Siloxane: Me₃Si-O-SiMe₂OMe, Me₃Si-O-SiMe₂OEt, EtOSiMe₂-O-SiMe₂OEt, Me₃Si-O-SiMe(OMe)-O-SiMe₃, Me₃Si-O-SiMe(OEt)-O-SiMe₃, MeO-SiMe₂-(O-SiMe₂)ₘ-O-SiMe₂-OMe und EtO-SiMe₂-(O-SiMe₂)ₘ-O-SiMe₂-OEt mit m = 1 bis 10, Me₃Si-O-(SiMe₂-O)ₙ (SiMe(OMe)-O)ₒ-SiMe₃ und Me₃Si-O-(SiMe₂-O)ₙ(SiMe(OEt)-O)ₒ-SiMe₃ mit n = 0 bis 10 und o = 1 bis 10.

Die Verbindung **D** kann auch eine Mischung verschiedener Verbindungen der allgemeinen Formeln **VI** sein, bei denen gegebenenfalls die Reste **R**¹³**, R**¹⁴ und **R**¹⁵ verschiedene Reste der allgemeinen Formel II sein können.

Ebenfalls Gegenstand der Erfindung ist ein Verfahren zur Hydrosilylierung der Mischung **M** enthaltend
Verbindung **A,** die mindestens ein direkt an Si gebundenes Wasserstoffatom enthält,
Verbindung **B,** die mindestens eine Kohlenstoff-Kohlenstoff-Mehrfachbindung enthält,
Verbindung **C,** die mindestens eine kationische Si(II)-Gruppierung enthält und
Verbindung **D,** die mindestens eine direkt an Silicium gebundene Alkoxygruppierung enthält,
bei dem die Mischung **M** erwärmt wird.

Bei dem Verfahren wird Verbindung **A** mit Verbindung **B** in Gegenwart von Verbindung **C** als Hydrosilylierungskatalysator umgesetzt. Die durch Verbindung **C** als nichtmetallischem Hydrosilylierungskatalysator katalysierte und durch Inhibitor D unterdrückte Hydrosilylierungsreaktion wird durch Temperaturerhöhung ausgelöst

Bei dem Verfahren wird die Mischung **M** auf mindestens 40°C und höchstens 150°C, besonders bevorzugt auf 50 bis 120°C, insbesondere auf 60 bis 100°C erwärmt.

Das molare Verhältnis der Verbindungen **A** und **B** beträgt bezogen auf die vorhandenen Si-H bzw. die ungesättigten Kohlenstoffgruppierungen bevorzugt mindestens 1:100 und höchstens 100:1, besonders bevorzugt mindestens 1:10 und höchstens 10:1, ganz besonders bevorzugt mindestens 1:2 und höchstens 2:1.

Das molare Verhältnis zwischen der Verbindung **C** und den in der Verbindung **A** vorhandenen Si-H-Gruppierungen beträgt bevorzugt mindestens 1:10⁷ und höchstens 1:1, besonders bevorzugt mindestens 1:10⁶ und höchstens 1:10, ganz besonders bevorzugt mindestens 1:10⁵ und höchstens 1:50.

Das molare Verhältnis der Verbindungen **C** und **D** beträgt bezogen auf die vorhandenen kationischen Si(II)-Gruppierung der Verbindung **C** zu den direkt an Silicium gebundenen Alkoxygruppierung der Verbindung **D** bevorzugt mindestens 1:1 und höchstens 1:200 besonders bevorzugt mindestens 1:5 und höchstens 1:100, ganz besonders bevorzugt mindestens 1:10 und höchstens 1:50.

Die Verbindungen **A, B, C** und **D** können bei niedriger Temperatur, insbesondere unterhalb von 30°C, insbesondere unterhalb von 20°C in beliebiger Reihenfolge vermischt werden, wobei das Vermischen in einer der Fachkraft bekannten Weise erfolgt. Bevorzugt sind Ausführungsformen, bei denen ein Gemisch aus **C** und **D** mit **A** oder mit **B** oder dem Gemisch aus **A** und **B** gemischt wird. Bei einer weiteren bevorzugten Ausführungsform wird **D** mit **A** oder mit **B** oder mit dem Gemisch aus **A** und **B** gemischt und anschließend **C** zugegeben.

In einer weiteren Ausführungsform wird die Verbindung **C** in einer der Verbindungen **A** oder **B** oder **D** oder in binären Gemischen aus **A,B** und **D** oder in dem ternären Gemisch aus **A, B** und **D** im Gemisch der beiden Verbindungen beispielsweise durch die oben beschriebene Protonierungsreaktion erzeugt.

Die Umsetzung der Verbindungen **A** und **B** in Gegenwart der Verbindungen **C** und **D** kann mit oder ohne Zusatz einer oder mehrerer Lösemittel durchgeführt werden. Der Anteil des Lösemittels oder des Lösemittelgemisches beträgt bezogen auf die Summe der Verbindungen **A** und **B** bevorzugt mindestens 0,1 Gew.-% und höchstens die 1000-fache Gewichtsmenge, besonders bevorzugt mindestens 10 Gew. % und höchstens die 100-fache Gewichtsmenge, ganz besonders bevorzugt mindestens 30 Gew. % und höchstens die 10-fache Gewichtsmenge.

Als Lösemittel können bevorzugt aprotische Lösemittel, beispielsweise Kohlenwasserstoffe wie Pentan, Hexan, Heptan, Cyclohexan oder Toluol, Chlorkohlenwasserstoffe wie Dichlormethan, Chloroform, Chlorbenzol oder 1,2-Dichlorethan, oder Nitrile wie z.B. Acetonitril oder Propionitril, eingesetzt werden.

Die Mischung **M** kann beliebige weitere Verbindungen wie z.B. Prozesshilfsstoffe, z.B. Emulgatoren, Füllstoffe, z.B. hochdisperse Kieselsäure oder Quarz, Stabilisatoren, z.B. Radikalinhibitoren, Pigmente, z.B. Farbstoffe oder Weißpigmente, z.B. Kreide oder Titandioxid enthalten.

Die Umsetzung kann unter Umgebungsdruck oder unter vermindertem oder unter erhöhtem Druck durchgeführt werden.

Der Druck beträgt bevorzugt mindestens 0,01 bar und höchstens 100 bar, besonders bevorzugt mindestens 0,1 bar und höchstens 10 bar, ganz besonders bevorzugt wird die Umsetzung bei Umgebungsdruck durchgeführt. Sind jedoch an der Umsetzung Verbindungen beteiligt, die bei der Reaktionstemperatur gasförmig vorliegen, erfolgt bevorzugt eine Umsetzung unter erhöhtem Druck, besonders bevorzugt bei dem Dampfdruck des Gesamtsystems.

Alle vorstehenden Symbole der vorstehenden Formeln weisen ihre Bedeutungen jeweils unabhängig voneinander auf. In allen Formeln ist das Siliciumatom vierwertig.

Es sind, falls jeweils nicht anders angegeben, alle Mengen- und Prozentangaben auf das Gewicht bezogen, und alle Temperaturen 20°C.

### Beispiel 1

Eine Mischung aus 239 mg (2,02 mmol) α-Methylstyrol und 300 mg (2,02 mmol) Pentamethyldisiloxan wird unter Inertgasatmosphäre (Argon) mit einer Lösung aus 1,8 mg (2,14 µmol) der Verbindung (π-Me₅C₅)Si⁺ B(C₆F₅)₄⁻ und 4,8 mg (0,041 mmol) Ethoxytrimethylsilan in 540 mg Dideuterodichlormethan unter Schütteln versetzt und bei 25 °C 8 Tage stehengelassen. Das Reaktionsgemisch wird nach dieser Standzeit NMR-spektroskopisch untersucht: α-Methylstyrol und Pentamethyldisiloxan sind in unveränderter Menge vorhanden, kein Hydrosilylierungsprodukt nachweisbar. Der Katalysator (π-Me₅C₅)Si⁺ B(C₆F₅)₄⁻ ist in unveränderter Menge NMR-spektroskopisch nachweisbar (Singulett bei δ = 2,2 ppm).

Anschließend wird die Probe 1 Std. 60°C erwärmt und dann erneut NMR-spektroskopisch untersucht: Bildung des Hydrosilylierungsprodukts, 1,1,1,3,3-Pentamethyl-3-(2-phenylpropyl) disiloxan, Umsatz > 99 %.

### Beispiel 2

Eine Mischung aus 239 mg (2,02 mmol) α-Methylstyrol und 300 mg (2,02 mmol) Pentamethyldisiloxan wird unter Inertgasatmosphäre (Argon) mit einer Lösung aus 1,7 mg (2,02 µmol) der Verbindung (π-Me₅C₅)Si⁺ B(C₆F₅)₄⁻ und 7,8 mg (0,041 mmol) Ethoxypentamethyldisiloxan in 860 mg Dideuterodichlormethan unter Schütteln versetzt und bei 25 °C 9 Tage stehengelassen. Das Reaktionsgemisch wird nach dieser Standzeit NMR-spektroskopisch untersucht: α-Methylstyrol und Pentamethyldisiloxan sind in unveränderter Menge vorhanden, kein Hydrosilylierungsprodukt nachweisbar. Der Katalysator (π-Me₅C₅)Si⁺ B(C₆F₅)₄⁻ ist in unveränderter Menge NMR-spektroskopisch nachweisbar (Singulett bei δ = 2,2 ppm).

Anschließend wird die Probe 1 Std. auf 60°C erwärmt und dann erneut NMR-spektroskopisch untersucht: Bildung des Hydrosilylierungsprodukts, 1,1,1,3,3-Pentamethyl-3-(2-phenylpropyl) disiloxan, Umsatz > 99 %.

### Beispiel 3

Eine Mischung aus 239 mg (2,02 mmol) α-Methylstyrol und 300 mg (2,02 mmol) Pentamethyldisiloxan wird unter Inertgasatmosphäre (Argon) mit einer Lösung aus 1,9 mg (2,26 µmol) der Verbindung (π-Me₅C₅)Si⁺ B(C₆F₅)₄⁻ und 7,5 mg (0,042 mmol) Dimethylphenylethoxysilan in 741 mg Dideuterodichlormethan unter Schütteln versetzt und bei 25 °C 8 Tage stehengelassen. Das Reaktionsgemisch wird nach dieser Standzeit NMR-spektroskopisch untersucht: α-Methylstyrol und Pentamethyldisiloxan sind in unveränderter Menge vorhanden, kein Hydrosilylierungsprodukt nachweisbar. Der Katalysator (π-Me₅C₅)Si⁺ B(C₆F₅)₄⁻ ist in unveränderter Menge NMR-spektroskopisch nachweisbar (Singulett bei δ = 2,2 ppm).

Anschließend wird die Probe 1 Std. auf 60°C erwärmt und dann erneut NMR-spektroskopisch untersucht: Bildung des Hydrosilylierungsprodukts, 1,1,1,3,3-Pentamethyl-3-(2-phenylpropyl) disiloxan, Umsatz > 99 %.

### Beispiel 4, nicht erfindungsgemäß

Analog Beispiel 1 unter Verwendung von B(C₆F₅)₃ anstelle von (π-Me₅C₅)Si⁺ B(C₆F₅)₄⁻

### Hydrosilylierung ohne Inhibitorzusatz

Eine Mischung aus 239 mg (2,02 mmol) α-Methylstyrol und 300 mg (2,02 mmol) Pentamethyldisiloxan wird unter Inertgasatmosphäre (Argon) mit einer Lösung aus 1,8 mg (2,14 µmol) der Verbindung (π-Me₅C₅) Si⁺ B(C₆F₅)₄⁻ in 540 mg Dideuterodichlormethan unter Schütteln versetzt und bei 25 °C 20 min. stehengelassen und nach dieser Zeit NMR-spektroskopisch untersucht. Die Hydrosilylierung unter Bildung des Hydrosilylierungsprodukts ist vollständig abgelaufen.

## Patentansprüche

1. Mischung **M** enthaltend
Verbindung **A,** die mindestens ein direkt an Si gebundenes Wasserstoffatom enthält,
Verbindung **B,** die mindestens eine Kohlenstoff-Kohlenstoff-Mehrfachbindungen enthält,
Verbindung **C,** die mindestens eine kationische Si(II)-Gruppierung enthält und
Verbindung **D,** die mindestens eine direkt an Silicium gebundene Alkoxygruppierung enthält, wobei das molare Verhältnis der Verbindungen **A** und **B** bezogen auf die vorhandenen Si-H bzw. die ungesättigten Kohlenstoffgruppierungen mindestens 1:100 und höchstens 100:1 beträgt, wobei ferner das molare Verhältnis zwischen der Verbindung **C** und den in der Verbindung **A** vorhandenen Si-H-Gruppierungen mindestens 1:10⁷ und höchstens 1:1 beträgt und wobei ferner das molare Verhältnis der Verbindungen **C** und **D** bezogen auf die vorhandenen kationischen Si(II)-Gruppierungen der Verbindung C zu den direkt an Silicium gebundenen Alkoxygruppierungen der Verbindung D mindestens 1:1 und höchstens 1:200 beträgt.

2. Verfahren zur Hydrosilylierung einer Mischung **M** nach Anspruch 1,
bei dem die Mischung **M** auf mindestens 40°C und höchstens 150°C erwärmt wird.

3. Mischung **M** nach Anspruch 1 oder Verfahren nach Anspruch 2, wobei die Verbindung **A** die allgemeine Formel I
**R¹R²R³Si-H** **(I)**
aufweist,
wobei die Reste **R**¹, **R**² und **R**³ unabhängig voneinander die Bedeutung Wasserstoff, Halogen, Silyloxyrest oder Kohlenwasserstoffrest haben, wobei jeweils einzelne Kohlenstoffatome durch Sauerstoffatome, Siliciumatome, Stickstoffatome, Halogen, Schwefel oder Phosphoratome ersetzt sein können.

4. Mischung **M** nach Anspruch 1 oder 3 oder Verfahren nach Anspruch 2 oder 3, wobei die Verbindung **B** ausgewählt wird aus Verbindungen der allgemeinen Formel **IIIa**
R⁴R⁵C=CR⁶R⁷ **(IIIa)**,
und aus Verbindungen der allgemeinen Formel **IIIb**
R⁸C≡CR⁹ **(IIIb)**,
wobei
**R⁴, R⁵, R⁶, R⁷, R⁸** und **R⁹** unabhängig voneinander linearen, verzweigten, acyclischen oder cyclischen, gesättigten oder einfach oder mehrfach ungesättigten C1-C20-Kohlenwasserstoffrest bedeuten, wobei einzelne Kohlenstoffatome durch Silicium, Sauerstoff, Halogen, Stickstoff, Schwefel oder Phosphor ersetzt sein können.

5. Mischung **M** nach Anspruch 1, 3 oder 4 oder Verfahren nach Anspruch 2, 3 oder 4, wobei die Verbindung **C** eine kationische Si(II)-Verbindung der allgemeinen Formel IV
**([Si(II)Cp]**⁺**)ₐ X^{a-}** **(IV)**
ist, worin
**Cp** ein π-gebundener Cyclopentadienylrest der allgemeinen Formel **V** ist, welcher mit den Resten **R^{y}** substituiert ist, 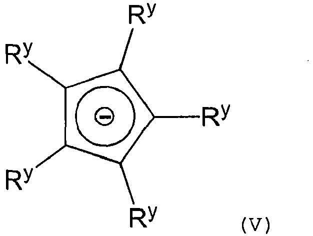
**R^{y}** einwertige Reste oder mehrwertige Reste sind, die zur Bildung anellierter Ringe auch miteinander verbunden sein können und
**X⁻** ein **a** wertiges Anion bedeutet, welches unter den Reaktionsbedingungen einer Hydrosilylierung mit dem kationischen Silicium(II)zentrum nicht reagiert.

6. Hydrosilylierbare Mischung **M** nach Anspruch 1, 3 oder 4 oder Verfahren nach Anspruch 2, 3 oder 4, wobei die Verbindung **C** ausgewählt wird aus den kationischen Si(II)-Verbindungen: 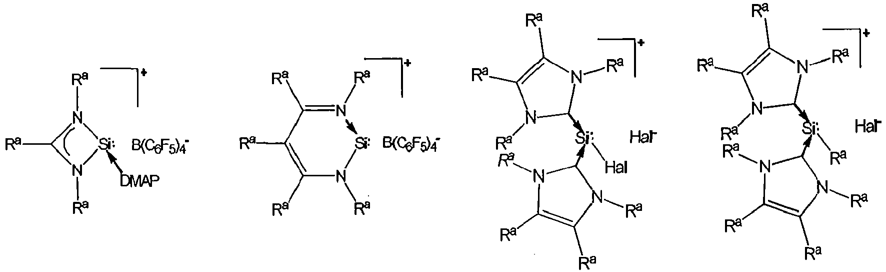 wobei die Reste **R^{a}** unabhängig voneinander Kohlenwasserstoffreste und **Hal** Halogen bedeuten.

7. Hydrosilylierbare Mischung **M** nach Anspruch 1, 3, 4, 5 oder 6 oder Verfahren nach Anspruch 2, 3, 4, 5 oder 6, wobei Verbindung **D** die allgemeine Formel **VI**
**R¹³R¹⁴R¹⁵Si-O-CH₂-R¹⁶** **(VI)**
aufweist, wobei die Reste **R¹³, R¹⁴** und **R¹⁵** unabhängig voneinander die Bedeutung Wasserstoff, Halogen, Silyloxyrest oder Kohlenwasserstoffrest haben, wobei jeweils einzelne Kohlenstoffatome durch Sauerstoffatome, Halogen, Schwefel oder Phosphoratome ersetzt sein können und
**R¹⁶** die Bedeutung Wasserstoff oder Kohlenwasserstoffrest haben, bei dem einzelne nicht benachbarte Kohlenstoffatome durch Sauerstoffatome, Silicium-, Halogen-, Schwefel- oder Phosphoratome ersetzt sein können.

## Claims

1. Mixture **M** containing
compound **A** which contains at least one hydrogen atom bound directly to Si,
compound **B** which contains at least one carbon-carbon multiple bond,
compound **C** which contains at least one cationic Si(II) group and
compound **D** which contains at least one alkoxy group bound directly to silicon, wherein the molar ratio of the compounds **A** and **B** based on the Si-H groups present and the unsaturated carbon groups is at least 1:100 and not more than 100:1, further wherein the molar ratio between the compound **C** and the Si-H groups present in the compound **A** is at least 1:10⁷ and not more than 1:1 and further wherein the molar ratio of the compounds **C** and **D** based on the cationic Si(II) groups present in the compound **C** to the alkoxy groups bound directly to silicon in the compound **D** is at least 1:1 and not more than 1:200.

2. Method for hydrosilylating a mixture **M** according to Claim 1,
wherein the mixture **M** is heated to at least 40°C and not more than 150°C.

3. Mixture **M** according to Claim 1 or method according to Claim 2, wherein the compound **A** has the general formula I
**R¹R²R³Si-H** **(I)**,
where the radicals **R¹, R²** and **R³** are each, independently of one another, hydrogen, halogen, a silyloxy radical or a hydrocarbon radical, in which individual carbon atoms can in each case be replaced by oxygen atoms, silicon atoms, nitrogen atoms, halogen, sulfur or phosphorus atoms.

4. Mixture **M** according to Claim 1 or 3 or method according to Claim 2 or 3, wherein the compound **B** is selected from among compounds of the general formula **IIIa**
R⁴R⁵C=CR⁶R⁷ **(IIIa)**,
and compounds of the general formula **IIIb**
R⁸C≡CR⁹ **(IIIb),**
where
**R⁴, R⁵, R⁶, R⁷, R⁸** and **R⁹** are each, independently of one another, a linear, branched, acyclic or cyclic, saturated or monounsaturated or polyunsaturated C1-C20-hydrocarbon radical, in which individual carbon atoms can be replaced by silicon, oxygen, halogen, nitrogen, sulfur or phosphorus.

5. Mixture **M** according to Claim 1, 3 or 4 or method according to Claim 2, 3 or 4, wherein the compound **C** is a cationic Si(II) compound of the general formula IV
**([Si(II)Cp]⁺)ₐ X^{a-}** **(IV)**
where
**Cp** is a π-bonded cyclopentadienyl radical of the general formula **V**, which is substituted by the radicals **R^{y}**, 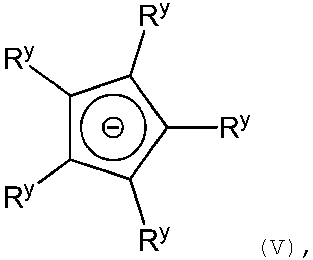 the radicals
**R^{y}** are monovalent radicals or polyvalent radicals which can also be joined to one another to form fused rings and
**X⁻** is an **a**-valent anion which does not react with the cationic silicon(II) center under the reaction conditions of a hydrosilylation.

6. Hydrosilylatable mixture **M** according to Claim 1, 3 or 4 or method according to Claim 2, 3 or 4, wherein the compound **C** is selected from among the cationic Si(II) compounds: 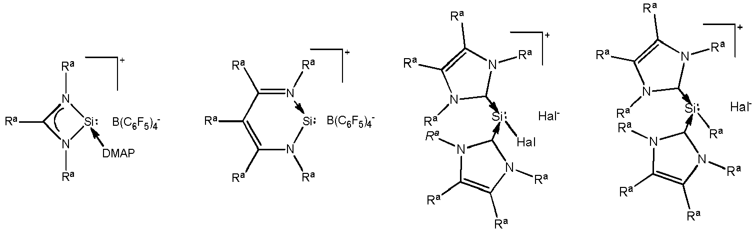 where the radicals **R^{a}** are, independently of one another, hydrocarbon radicals and **Hal** is halogen.

7. Hydrosilylatable mixture **M** according to Claim 1, 3, 4, 5 or 6 or method according to Claim 2, 3, 4, 5 or 6, wherein compound **D** has the general formula **VI**
**R¹³R¹⁴R¹⁵Si-O-CH₂-R¹⁶** **(VI)**
where the radicals **R¹³, R¹⁴** and **R¹⁵** are each, independently of one another, hydrogen, halogen, a silyloxy radical, or a hydrocarbon radical, in which individual carbon atoms can in each case be replaced by oxygen atoms, halogen, sulfur or phosphorus atoms, and
**R**¹⁶ is hydrogen or a hydrocarbon radical in which individual nonadjacent carbon atoms can be replaced by oxygen atoms, silicon, halogen, sulfur or phosphorus atoms.

## Revendications

1. Mélange **M,** contenant :
un composé **A,** qui contient au moins un atome d'hydrogène relié directement à Si,
un composé **B,** qui contient au moins une liaison multiple carbone-carbone,
un composé **C,** qui contient au moins un groupement Si(II) cationique, et
un composé **D,** qui contient au moins un groupement alcoxy relié directement au silicium, le rapport molaire entre les composés **A** et **B** par rapport aux Si-H présents ou aux groupements carbonés insaturés étant d'au moins 1:100 et d'au plus 100:1, le rapport molaire entre le composé **C** et les groupements Si-H présents dans le composé **A** étant par ailleurs d'au moins 1:10⁷ et d'au plus 1:1, et le rapport molaire entre les composés **C** et **D** par rapport aux groupements Si(II) cationiques du composé C présents et aux groupements alcoxy directement reliés au silicium du composé D étant d'au moins 1:1 et d'au plus 1:200.

2. Procédé d'hydrosilylation d'un mélange **M** selon la revendication 1, selon lequel le mélange **M** est porté à au moins 40 °C et au plus 150 °C.

3. Mélange **M** selon la revendication 1 ou procédé selon la revendication 2, dans lequel le composé **A** présente la formule générale I :
**R¹R²R³Si-H** **(I)**
dans laquelle les radicaux **R¹, R²** et **R³** ont indépendamment les uns des autres la signification hydrogène, halogène, radical silyloxy ou radical hydrocarboné, des atomes de carbone individuels pouvant à chaque fois être remplacés par des atomes d'oxygène, des atomes de silicium, des atomes d'azote, des atomes d'halogène, de soufre ou de phosphore.

4. Mélange **M** selon la revendication 1 ou 3 ou procédé selon la revendication 2 ou 3, dans lequel le composé **B** est choisi parmi les composés de la formule générale **IIIa** :
R⁴R⁵C=CR⁶R⁷ **(IIIa)**,
et parmi les composés de la formule générale **IIIb** :
R⁸C≡CR⁹ **(IIIb),**
dans lesquelles **R⁴, R⁵, R⁶, R⁷, R⁸** et **R⁹** signifient indépendamment les uns des autres un radical hydrocarboné en C1-C20 linéaire, ramifié, acyclique ou cyclique, saturé ou mono- ou polyinsaturé, dans lequel des atomes de carbone individuels peuvent être remplacés par silicium, oxygène, halogène, azote, soufre ou phosphore.

5. Mélange **M** selon la revendication 1, 3 ou 4, ou procédé selon la revendication 2, 3 ou 4, dans lequel le composé **C** est un composé de Si(II) cationique de la formule générale IV :
**([Si(II)Cp]⁺)ₐ X^{a-}** **(IV)**
dans laquelle
**Cp** est un radical cyclopentadiényle à liaison π de la formule générale **V,** qui est substitué avec les radicaux **R^{y},** 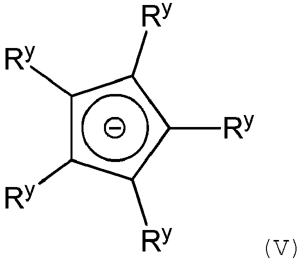
les **R^{y}** sont des radicaux monovalents ou des radicaux polyvalents, qui peuvent également être reliés les uns avec les autres pour former des cycles annelés, et
**X⁻** signifie un anion **a**-valent, qui ne réagit pas dans les conditions de réaction d'une hydrosilylation avec le centre silicium (II) cationique.

6. Mélange hydrosilylable **M** selon la revendication 1, 3 ou 4, ou procédé selon la revendication 2, 3 ou 4, dans lequel le composé **C** est choisi parmi les composés de Si(II) cationiques : 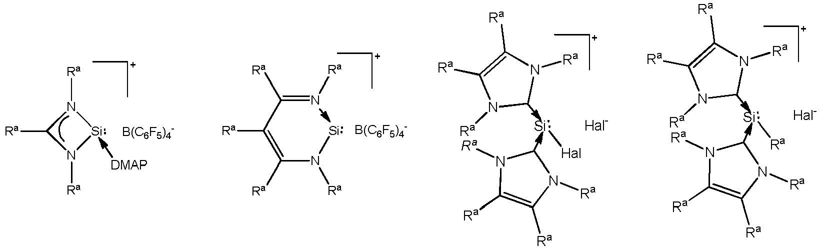 les radicaux **R^{a}** signifiant indépendamment les uns des autres des radicaux hydrocarbonés, et **Hal** signifiant halogène.

7. Mélange hydrosilylable **M** selon la revendication 1, 3, 4, 5 ou 6, ou procédé selon la revendication 2, 3, 4, 5 ou 6 dans lequel le composé **D** présente la formule générale **VI** :
**R¹³R¹⁴R¹⁵Si-O-CH₂-R¹⁶** **(VI)**
dans laquelle les radicaux **R¹³**, **R¹⁴** et **R¹⁵** ont indépendamment les uns des autres la signification hydrogène, halogène, radical silyloxy ou radical hydrocarboné, des atomes de carbone individuels pouvant à chaque fois être remplacés par des atomes d'oxygène, des atomes d'halogène, de soufre ou de phosphore, et
**R¹⁶** a la signification hydrogène ou radical hydrocarboné, dans lequel des atomes de carbone non voisins individuels peuvent être remplacés par des atomes d'oxygène, des atomes de silicium, d'halogène, de soufre ou de phosphore.
